# EUROPEAN PATENT SPECIFICATION

(11) **EP 1 942 953 B1**
(45) Date of publication and mention of the grant of the patent: **03.02.2010**
(21) Application number: 06850407.5
(22) Date of filing: 31.10.2006
(51) Int. Cl.: A61L 2/18, A61L 2/26

(54) **SPOUT ASSEMBLY FOR ENDOSCOPE REPROCESSOR**
AUSLAUFANORDNUNG FÜR EINEN ENDOSKOP-REPROZESSOR
ENSEMBLE GOULOTTE POUR DISPOSITIF DE RETRAITEMENT D'ENDOSCOPE

(30) Priority: 02.11.2005 US 264902
(43) Date of publication of application: 16.07.2008
(73) Proprietor: Minntech Corporation, Minneapolis, Minnesota 55447-4822 (US)
(72) Inventor: DEPREY, Eric J., Minneapolis, MN 55419 (US); WHITELEY, Gregory Stuart, Queenscliff, NSW 2096 (AU)
(74) Representative: Liesegang, Eva
(86) International application number: PCT/US2006/060393
(87) International publication number: WO 2007/089359

(56) References cited:
- CA-A1- 2 286 528
- DE-A1- 4 306 334
- DE-A1-102004 048 900
- US-A1- 2003 173 423
- US-A1- 2005 220 665

## Description

### BACKGROUND OF THE INVENTION

The present invention relates to the field of Automatic Endoscope Reprocessors (hereinafter AERs) and similar equipment having similar characteristics and requirements.

Such equipment often is connected to a shared or community potable water supply to provide the AER with water for washing, rinsing, and diluting liquid chemicals for disinfecting endoscopes during reprocessing.

The present invention defined by claim 1 relates more particularly to water and disinfectant delivery to one or more basins of the reprocessor. In the past, various forms of spouts or other water delivery devices were used to deliver potable water from a permanently connected public water supply.

To protect the sanitary integrity of the public water supply, governmental units have promulgated various standards which must be met by equipment (including endoscope reprocessors) sold for use within the territory under the supervision of that respective governmental unit. One such standard is European Standard EN 1717:2000, Protection against pollution of potable water in water installations and general requirements of devices to prevent pollution by backflow. This European Standard is given the status of a national standard by those European countries which either publish an identical text or endorse the European Standard itself.
US 2003/0173423 A1 discloses a water tool having a jet spray mode of operation. The water tool is a part of a pull out spout of a faucet. In the embodiment shown, the faucet is mounted next to a kitchen sink such that the projection of the spout falls into the periphery of the sink when the spout is in the completely pulled-in position.
DE 102004048900 A1, which has been published after the priority date of the present application, discloses a water faucet for bath tubs or sinks comprising a faucet body having at least one opening for water intake and another opening for water outlet. Downstream of the water outlet's opening a channel is shown which redirects the flow of the water leaving the outlet opening.
US 2005/0220665 A1 discloses a method and apparatus for low temperature sterilization and disinfections to be applied to medical apparatus or instruments such as endoscopes. The apparatus comprises a reprocessor basin to which water is introduced through a top water inlet nozzle lying inside the periphery of the basin. In addition, chemical reagents such as disinfectants can be pumped from a chemical reagent tank through an inlet nozzle into the basin. Again, the spout of the chemical reagent inlet nozzle lies within the periphery of the basin.

### SUMMARY OF THE INVENTION

The present invention meets the requirements of a type AA unrestricted air gap under the above standard, and in addition, enables mixing of water with one or more other chemical components (typically liquid) of a disinfectant solution used in the basin of an endoscope reprocessor.

The present invention may include an apparatus for delivering water to an endoscope reprocessing basin while avoiding interference with a working area of the basin. In one aspect, the invention may have at least one spout selectively discharging a liquid in a generally vertically downward direction and also has a flow diverter spaced apart from the spout by an air gap, while being positioned under the spout to receive the liquid discharged by the spout. The flow diverter then redirects the discharged liquid from the vertical direction into the basin. Stated another way, the flow diverter is a surface redirecting the discharged liquid in a direction having a horizontal component and a vertical component.

The air gap preferably has a length equal to a predetermined minimum distance between the spout and the flow diverter. The spout preferably has a downwardly directed outlet and the flow diverter will have an upper edge. The length of the air gap may be more precisely defined to be between the outlet of the spout and the upper edge of the flow diverter.

The spout has a characteristic diameter, typically an inside diameter, and the length of the air gap is preferably equal to or greater than three times the diameter of the spout. ,

The invention may also include a feed pipe upstream of and in fluid communication with the spout. In this aspect, the feed pipe has a characteristic diameter, typically an inside diameter, and the length of the air gap may be equal to or greater than three times the diameter of the feed pipe.

In another aspect, the present invention may include a means for discharging a second liquid into the liquid from the spout in the region of the flow diverter such that the liquids are mixed.

In another aspect, the present invention may be seen to be a method of preventing backflow and directing a liquid into an endoscope reprocessor basin comprising the steps of discharging a liquid from a spout in a downward direction; receiving the liquid discharged from the spout in a flow diverter spaced apart from the spout; and redirecting the liquid in a predetermined direction having a substantial horizontal component.

### Brief Description of the Drawings

Figure 1 is a very diagrammatic view in perspective of a prior art Automatic Endoscope Reprocessor useful in the practice of the present invention.

Figure 2 is a plan view of a prior art rack with an endoscope therein suitable for use with the apparatus of Figure 1.

Figure 3 is a perspective view of the basins of the AER of Figure 1.

Figure 4 is a top plan view of the basins of Figure 3.

Figure 5 is an elevation view in section taken along line 5-5 of Figure 4.

Figure 6 is a fragmentary elevation view taken along line 6-6 of Figure 4.

Figure 7 is a perspective view of the spout assembly of the present invention showing the discharge and deflection of liquid from the assembly.

Figure 8 is an exploded view of the parts from Figure 7.

Figure 9 is a top plan view of a base useful in the practice of the present invention.

Figure 10 is a bottom plan view of the base of Figure 9.

Figure 11 is a perspective view from above of the base of Figure 9.

Figure 12 is a perspective view from below of the base of Figure 9.

Figure 13 is a front elevation view of the base of Figure 9.

Figure 14 is a section view of the base of Figure 9 taken along line 14-14 of Figure 10.

Figure 15 is a partially cut away section view of the base of Figure 9 along line 15-15 of Figure 10.

Figure 16 is a side view of a spout assembly showing an alternative embodiment of the present invention.

Figure 17 is a front elevation view of the alternate embodiment of Figure 16.

Figure 18 is a top plan view of the alternate embodiment of Figure 16.

### Detailed Description of the Invention

One example of a system for cleaning, disinfecting and/or drying endoscopes is shown in United States Patent 6,641,781 B2, issued November 4, 2003.

Another example of a device and method for cleaning and/or disinfecting endoscopes is shown in United States Patent 6,260,560 B1, issued July 17, 2001.

Still another example of a device and method for cleaning and/or disinfecting endoscopes is shown in European Patent Application EP 0 709 056 A1, published 01.05.1996.

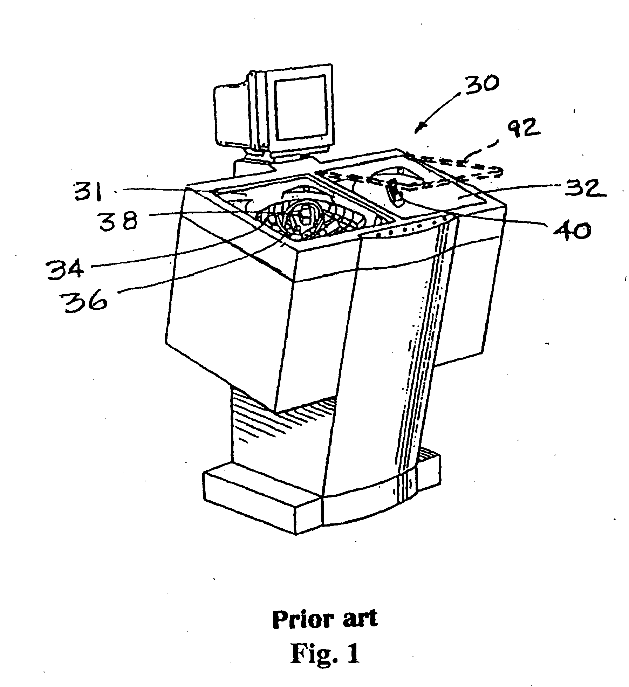

Referring now to the Figures, and most particularly to Figure 1, a disinfecting device or Automatic Endoscope Reprocessor (or AER) 30 may be seen. The disinfecting device 30 is provided with two trays or basins 31 and 32 in which a rack 34 is, with an endoscope 36 therein, can be accommodated. In Figure 1, a rack of this nature is located in the left hand tray. Both trays 31 and 32 are provided with a counter-connection block 40 which, when a rack 34 is placed in the tray 31, 32, can be connected to the connection block 38 arranged in rack 34. The counter-connection block 40 in the right hand tray can be seen in Figure 1. Each tray or basin 31, 32 may be provided with a lid 92, which may be transparent.

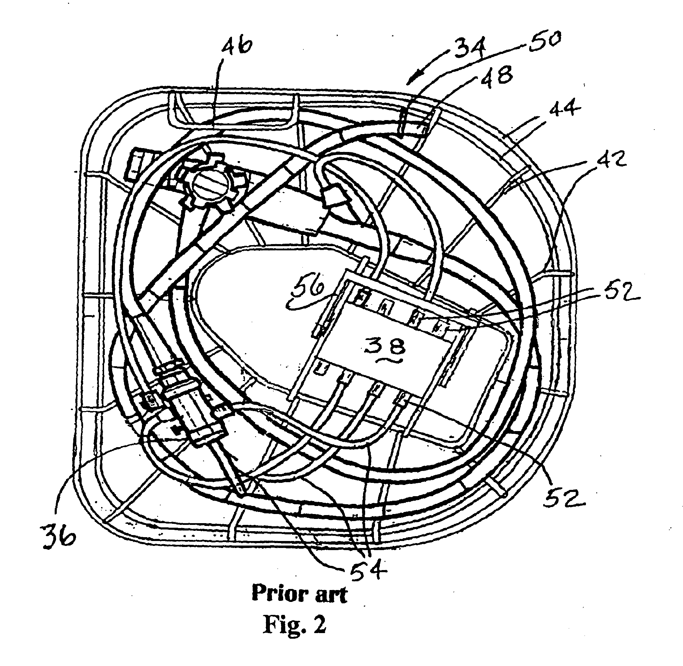

Referring now also to Figure 2, the rack 34 may be formed from bent rods 42 and 44 which are fixedly connected to one another. The rack 34 is provided with one or two handles 46, by means of which the rack can be gripped and lifted up. The rack 34 is furthermore formed in such a manner that an endoscope 36 can be placed therein in a more or less folded state. In order to be able to fix in particular the fragile end 48 of the endoscope, the rack may be provided with a tip holder 50.

The connection block 38 is arranged fixedly in the rack. This connection block is provided with passages and ports 52 which are connected to the passages and can be connected to the passages of the endoscope 36 by means of flexible tubes 54. On its underside (not visible in Figure 2), the connection block 38 is provided with connection points for the connection of counter-connection blocks 40 in either basin 31 or 32 of device 30. The connection block 38 is furthermore provided with a handle 56. By moving the handle 56, the connection block 38 can be connected to a counter-connection block 40 or removed therefrom.

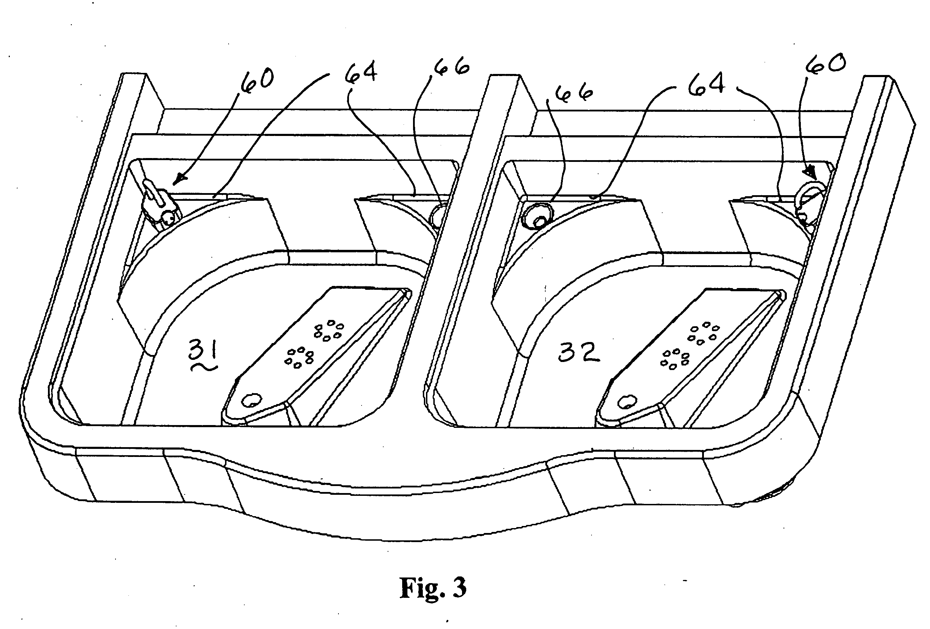

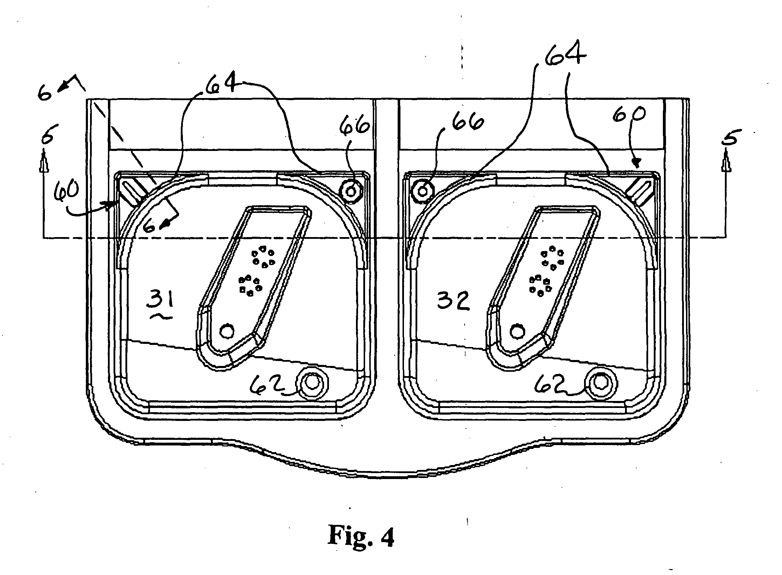

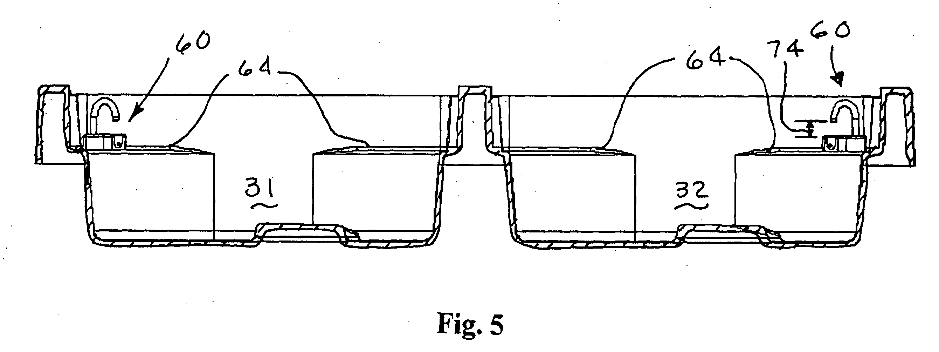

Referring now to Figures 3, 4 and 5, various views of the basins 31, 32 of the reprocessor 30 may be seen. Each basin preferably has a spout assembly 60 installed in one rear corner. It may be noted (particularly with reference to Figure 4) that the spout assembly 60 provides clearance for insertion of the rack 34 into the basin 31 or 32 without interference, because the spout assembly does not extend into the periphery of the basin and overlap the area where the rack 34 would extend when lowered into the basin. Each basin has a main drain 62 near the front thereof, and a pair of shelves 64 at the rear thereof, with one shelf supporting the spout assembly 60 and the other shelf containing an overflow drain 66.

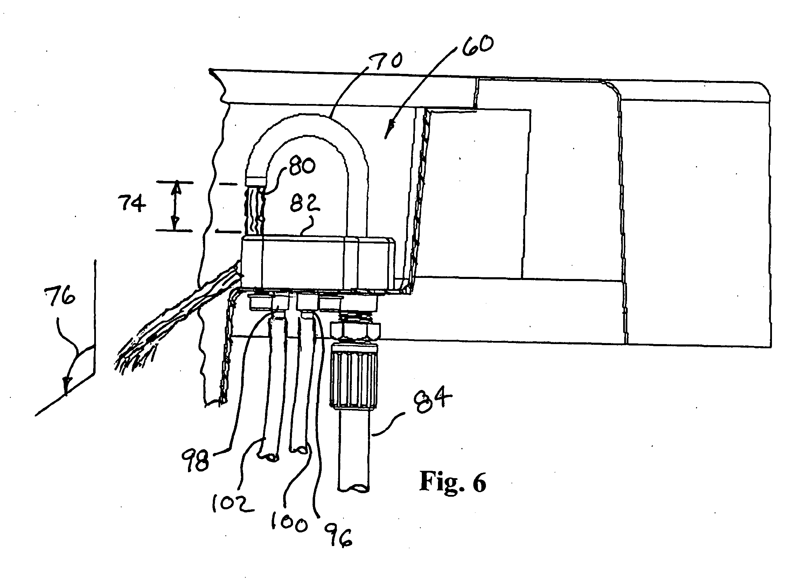

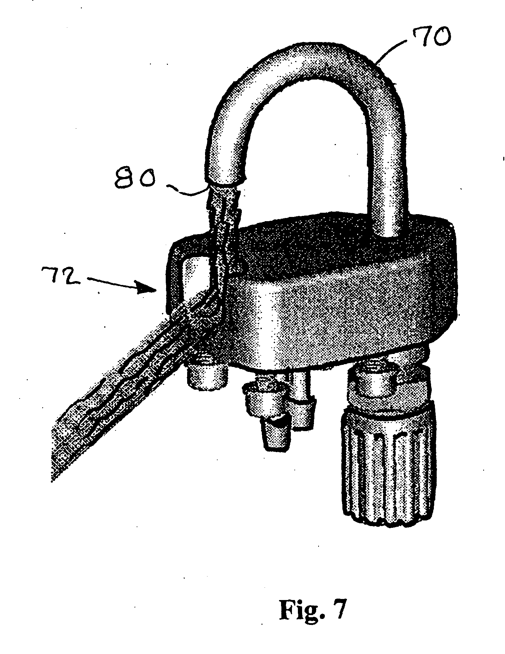

Referring now to Figures 6, 7 and 8, various details of the spout assembly or apparatus 60 may be seen. Spout assembly 60 preferably has a spout 70 for selectively discharging a liquid (typically potable water from a public water supply) in a generally vertically downward direction.

Spout assembly 60 also includes a flow diverter 72 spaced apart from the spout 70 by an air gap 74 and positioned under the spout and receiving the liquid discharged by the spout 70. The flow diverter 72 redirects the discharged liquid in a direction other than vertical to direct the discharged liquid into the basin. In the embodiment shown in Figures 6-8, the discharged liquid is initially directed at an angle 76 of about 125 degrees from the vertical, although it is to be understood that other angles are within the scope of the present invention.

In this embodiment, the flow diverter 72 is a surface 78 that may be seen to be redirecting the discharged liquid in a direction having a horizontal component and a vertical component. The air gap 74 is to be understood to have a length equal to a predetermined minimum distance between the spout and the flow diverter, with the predetermined minimum distance preferably equal to or greater than three or more diameters of the spout, more particularly either the inside or outside diameter of a discharge end or outlet 80 of the spout 70. It may thus be seen that the spout 70 has downwardly directed outlet 80 and the length of the air gap 74 is preferably measured between the outlet 80 of the spout and the flow diverter 72.
More precisely, in this embodiment the flow diverter 72 is preferably formed (in this embodiment) in a base 86 which has an upper edge 82 and the length of the air gap 74 is measured between the outlet 80 of the spout 70 and the upper edge 82 of the flow diverter 72.

The spout assembly 60 is preferably connected to a feed pipe 84 upstream of and in fluid communication with the spout 70. The feed pipe 84 has a diameter, and the length of the air gap 74 may be made equal to or greater than three times the diameter of the feed pipe 84 in the practice of the present invention. Furthermore, the portion of the spout 70 upstream of the outlet 80 may be considered the feed pipe for air gap dimensioning in accordance with the practice of the present invention. In that regard, the diameter used for dimensioning the air gap may be an inside or outside diameter of the feed pipe, including spout 70. It is preferred that the direction of discharge from the outlet 80 of spout 70 is not more than fifteen degrees from the vertical.

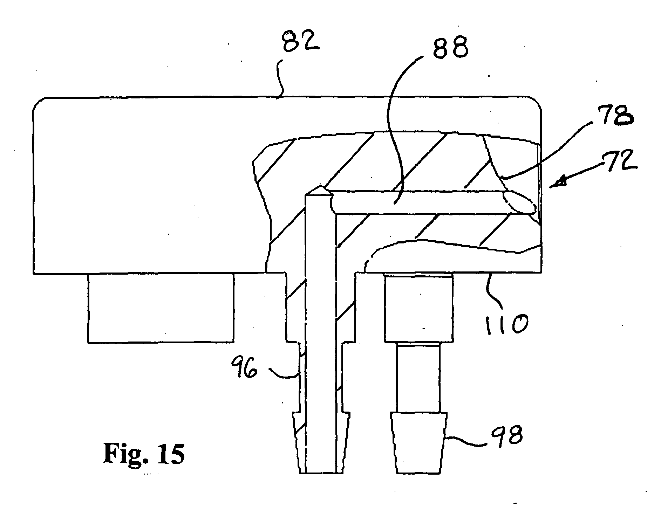

The liquid discharged from the spout 70 may be considered a first liquid and the apparatus of the present invention further includes means for discharging at least a second liquid (and preferably a third liquid) into the first liquid in the region of the flow diverter 72 such that the liquids are mixed. In the embodiment shown in Figures 6-8, the means for discharging the second liquid (and optionally the third liquid) is one or more internal passages 88 in the base 86 (shown in Figure 15).

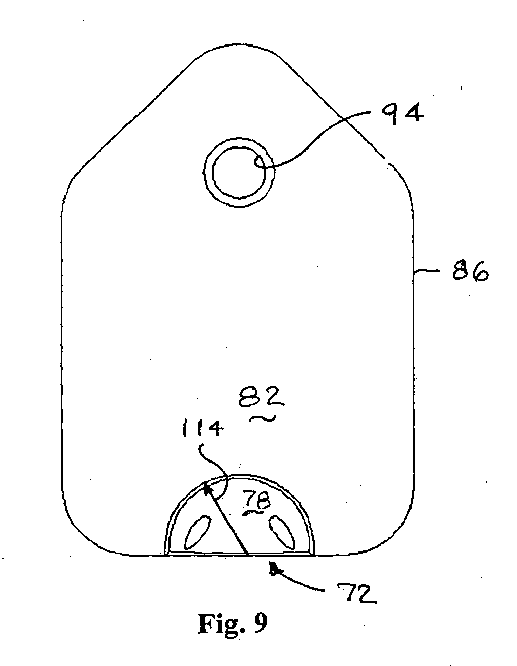

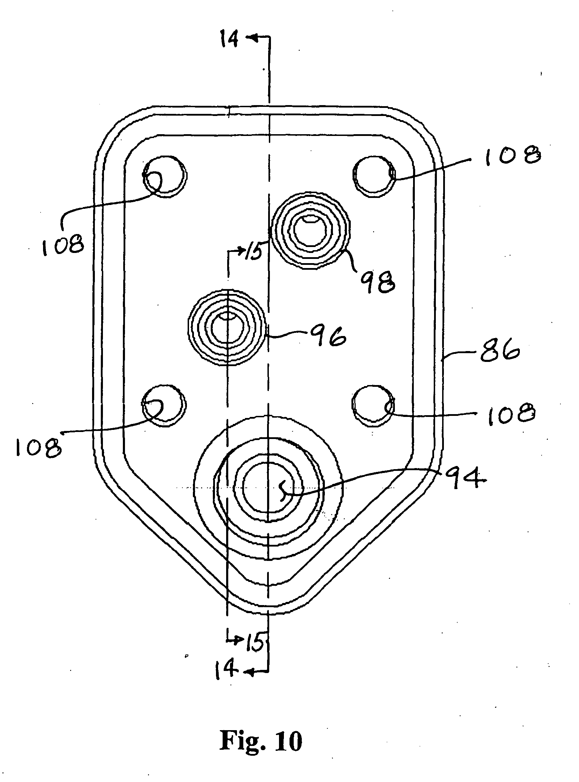

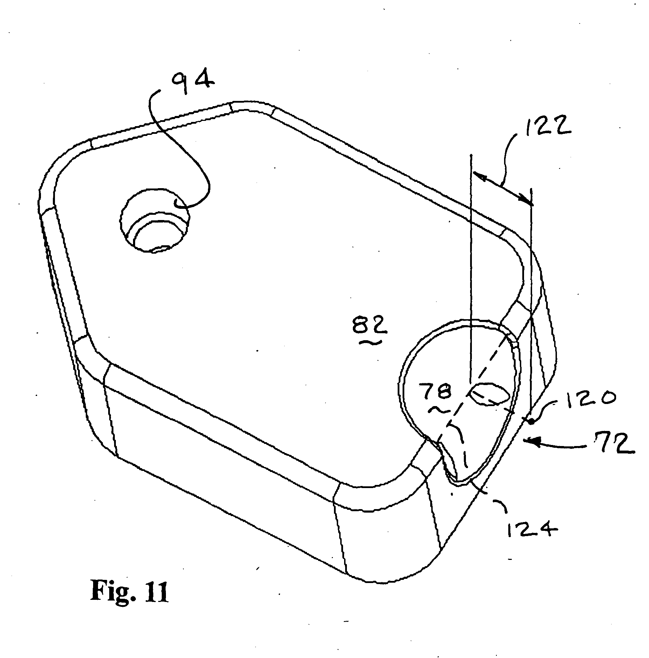

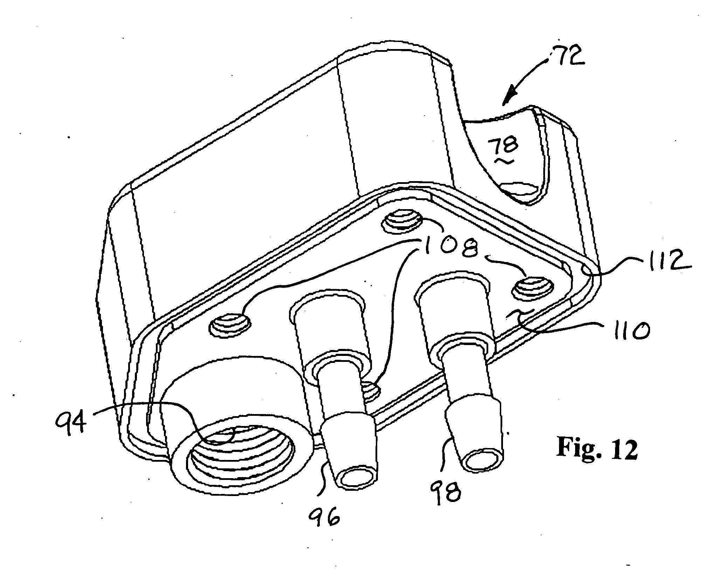

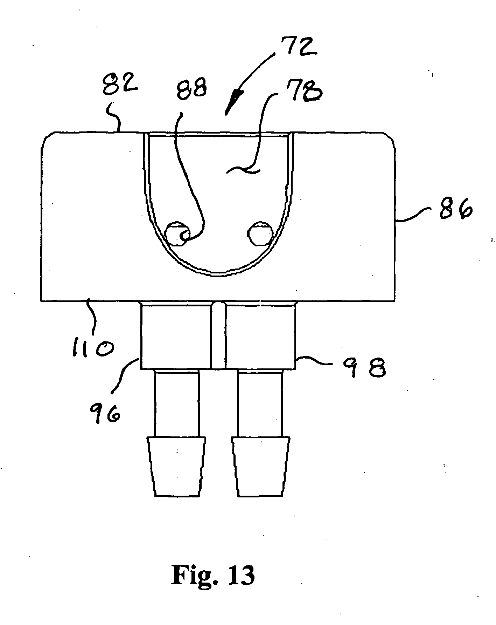

Referring now most particularly to Figures 9 through 15, various views of the base 86 for the spout assembly 60 of the present invention may be seen. It is to be understood that alternative embodiments for the base 86 and for the flow diverter 72 are within the scope of the present invention, and the base 86 and flow diverter 72 are shown and described as an illustration of one embodiment of the present invention. Base 86 is shown as a solid material formed of, for example 316 stainless steel, but the base 86 (and other components of the spout assembly 60) may advantageously be formed of other suitable materials, such as other metals or polymers, if desired, and the flow diverter 72 may be formed, for example, from a sheet of material, if desired.

Base 86 has a through bore 94 to permit the passage of water to spout 70. In this embodiment, base 86 also has fittings 96 and 98 to receive respective tubes 100 and 102 to couple the two liquid disinfectant chemicals to the base and to their respective internal passages 88.

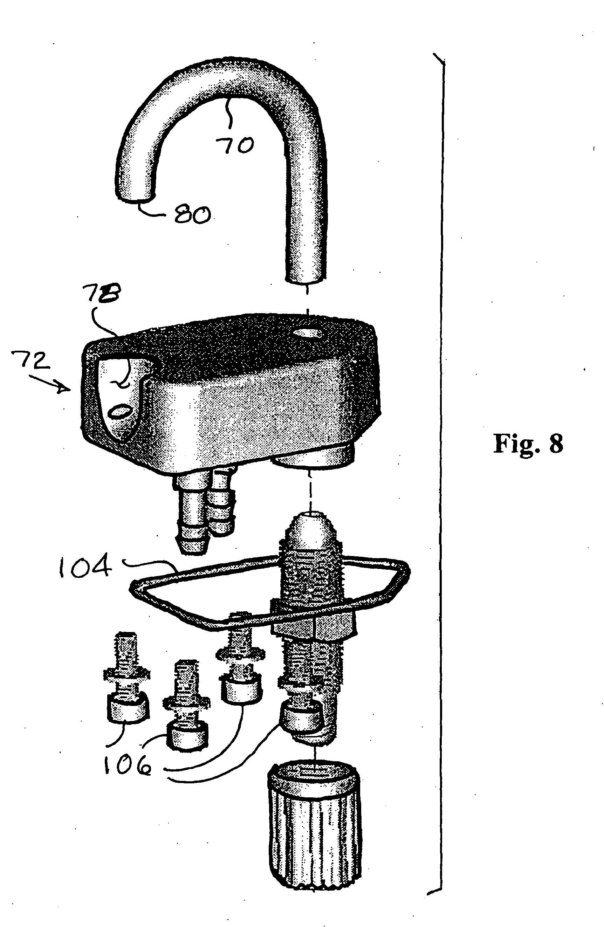

Referring now again to Figure 8, an O-ring 104 may be used to seal the base 86 to shelf 64 and a plurality of fasteners 106, such as cap screws and washers, may be used to secure base 86 to shelf 64, by receiving the fasteners 106 into blind threaded bores 108 in the bottom surface 110 of base 86. A groove 112 may be formed in the bottom surface 110 to receive O-ring 104 or another seal may be used to seal the base to the shelf, if desired.

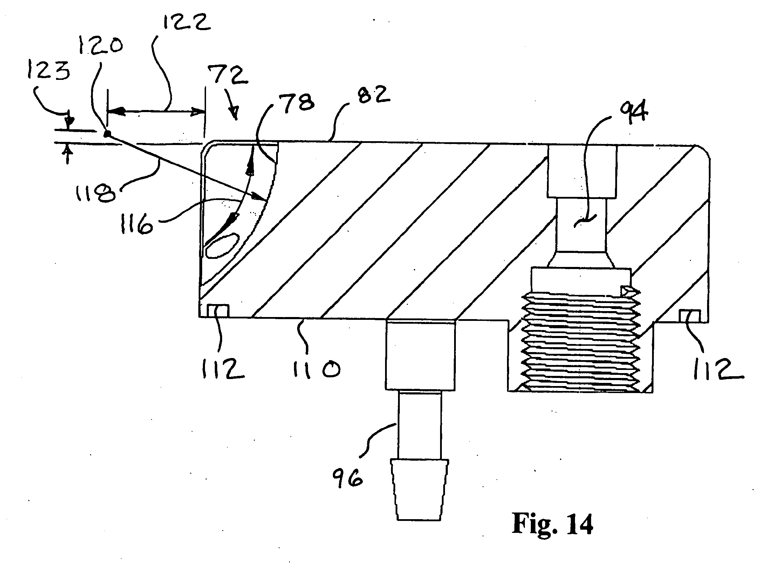

It is to be understood that the surface contour of the flow diverter 72 can take many forms while still remaining within the scope of the present invention. Referring now again to Figures 9, 11 and 14, one example surface 78 may be formed by a cutout having a radius 114 of 8 mm (shown in Figure 9) which is swept through an arc 116 (shown in Figure 14) having a radius 118 of 40 mm from a center 120 located a horizontal distance 122 of 29.92 mm and a vertical distance 123 of 2.55 mm from the front edge 124 of the envelope of top edge 82 of the base 86.

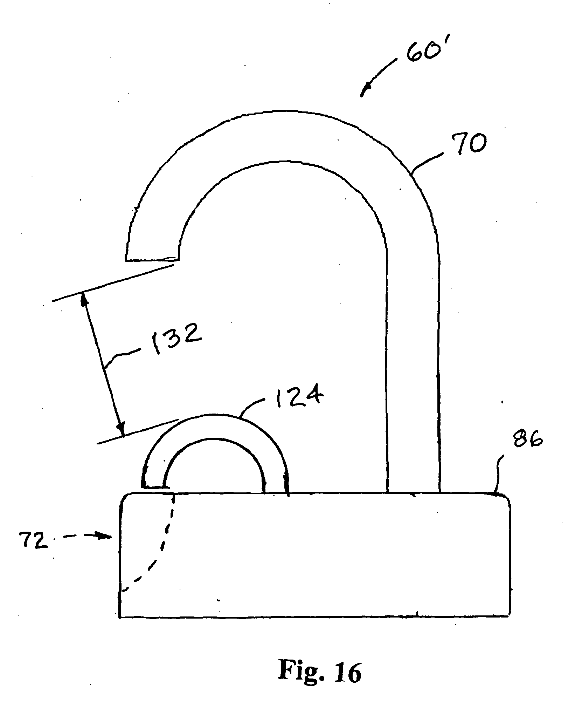

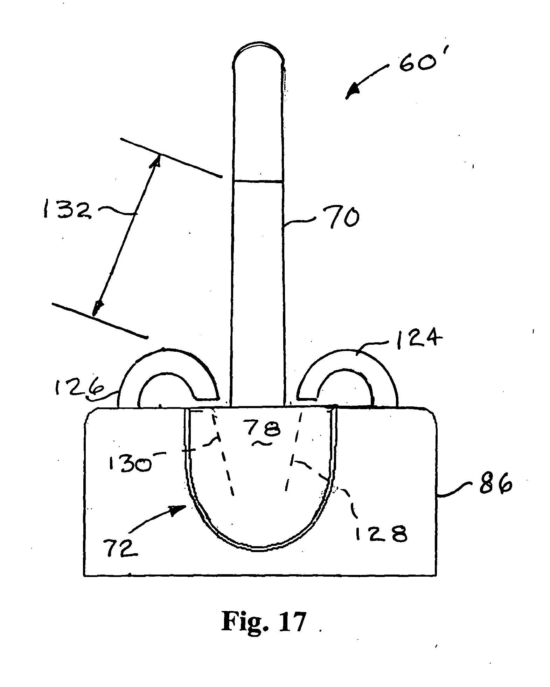

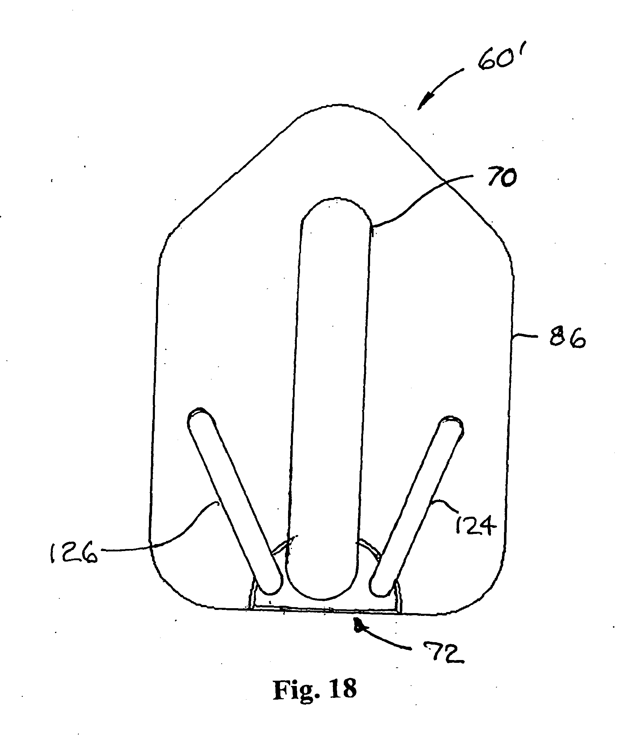

Referring now to Figures 16, 17 and 18, an alternative embodiment 60' of the spout assembly of the present invention may be seen. In this embodiment, the water spout 70 is the same or similar to that in the previous embodiment, but internal passages 88 are omitted and replaced by liquid chemical delivery spouts 124, 126. The flow diverter 72 may be the same or similar to that in the previous embodiment, except that there are no apertures for the internal passages 88 in the surface 78 of the flow diverter 72 in this embodiment. The spouts 124 and 126 each have internal passages for delivery of a liquid chemical. Each of spouts 124 and 126 may be directed vertically downward or may be directed at an angle indicated by dashed lines 128, 130 to foster mixing with the stream of water from the spout 70. It is preferable in this embodiment that the chemical spouts 124 and 126 are each spaced a distance 132 equal to or greater than at least three diameters of the water spout 70 away from the water spout 70.

## Claims

1. Endoscope reprocessor (30) comprising an endoscope reprocessing basin (31, 32) and an apparatus (60, 60') for delivering water to said endoscope reprocessing basin (31, 32) while avoiding interference with a working area of the basin (31, 32), the apparatus is **characterised by**
a. a spout (70) selectively discharging a liquid in a vertically downward direction; and
b. a flow diverter (72) spaced apart from the spout (70) by an air gap (74) and positioned under the spout (70) and receiving the liquid discharged by the spout (70) and redirecting the discharged liquid in a direction other than vertical into the endoscope basin (31, 32).

2. The endoscope reprocessor (30) of claim 1 wherein the flow diverter comprises a surface (78) redirecting the discharged liquid in a direction having a horizontal component and a vertical component.

3. The endoscope reprocessor (30) of claim 1 wherein the air gap (74) further comprises a length equal to a predetermined minimum distance between the spout (70) and the flow diverter (72), wherein the spout (70) has a downwardly directed outlet (80) and a diameter and the flow diverter (72) has an upper edge,
wherein the length of the air gap (74) is measured between the outlet (80) of the spout (70) and the upper edge of the flow diverter (72),
and wherein the length of the air gap is equal to or greater than three times the diameter of the spout.

4. The endoscope reprocessor (30) of claim 3 further comprising a feed pipe (84) upstream of and in fluid communication with the spout and wherein the feed pipe has a diameter and the length of the air gap is equal to or greater than three times the diameter of the feed pipe.

5. The endoscope reprocessor (30) of claim 1 wherein the liquid discharged from the spout comprises a first liquid and the apparatus further includes
c. means (88, 96, 124) for discharging a second liquid into the first liquid in the region of the flow diverter (72) such that the first and second liquids are mixed.

6. The endoscope reprocessor (30) of claim 5 further comprising:
d. means (88, 98, 126) for discharging a third liquid into the first liquid in the region of the flow diverter (72) such that the first, second and third liquids are mixed.

7. The endoscope reprocessor (30) of claim 1 wherein the direction of discharge of the spout (70) is not more than fifteen degrees from the vertical.

8. The endoscope reprocessor (30) of claim 1
wherein the flow diverter (72) has a concave surface (78) redirecting the stream.

9. The endoscope reprocessor (30) of claim 8 further including at least one passage (88, 124, 126) for delivering a liquid chemical to the flow diverter (72) to mix the at least one liquid chemical with the stream of water.

10. The endoscope reprocessor (30) of claim 9 wherein the at least one passage (88) exits in a surface (78) of the flow diverter (72).

11. The endoscope reprocessor (30) of claim 9 wherein the at least one passage is in at least one liquid chemical delivery spout (124) above the flow diverter (72).

12. The endoscope reprocessor (30) of claim 11 wherein the at least one chemical delivery spout (124, 126) emits a stream at an angle towards the stream of water.

13. The endoscope reprocessor (30) of claim 11 wherein the at least one chemical delivery spout (124, 126) is spaced a distance apart from the water spout (70) equal to or greater than three times the diameter of the water spout.

14. The endoscope reprocessor (30) of claim 3 or 13 wherein the diameter is an inside diameter or an outside diameter.

15. A method of preventing backflow and directing a liquid into an endoscope reprocessor basin (31, 32) comprising the steps of:
a. discharging a liquid from a spout (70) in a downward direction;
b. receiving the liquid discharged from the spout (70) in a flow diverter (72) spaced apart from the spout (70); and
c. redirecting the liquid in a predetermined direction having a substantial horizontal component into the endoscope reprocessor basin (31, 32).

## Patentansprüche

1. Endoskop-Wiederaufbereitungseinrichtung (30), umfassend ein Endoskop-Wiederaufbereitungseinrichtungs-Becken (31, 32) und eine Vorrichtung (60, 60') zum Einlassen von Wasser in das Endoskop-Wiederaufbereitungseinrichtungs-Becken (31, 32) unter Vermeidung einer Behinderung eines Arbeitsbereichs des Beckens (31, 32), wobei das Gerät durch folgendes **gekennzeichnet** ist:
a. einen Auslass (70), der eine Flüssigkeit selektiv in eine Richtung vertikal nach unten ausgibt; und
b. einen Fluss-Umlenker (72), der von dem Auslass (70) durch einen Luftspalt (74) getrennt ist, unter dem Auslass (70) angeordnet ist und die von dem Auslass (70) ausgelassene Flüssigkeit empfängt und die ausgelassene Flüssigkeit in eine andere Richtung als die vertikale Richtung in das Endoskop-Becken (31, 32) umlenkt.

2. Endoskop-Wiederaufbereitungseinrichtung (30) nach Anspruch 1, bei der der Fluss-Umlenker eine Oberfläche (78) umfasst, die die ausgelassene Flüssigkeit in eine Richtung umlenkt, die eine horizontale und eine vertikale Komponente hat.

3. Endoskop-Wiederaufbereitungseinrichtung (30) nach Anspruch 1, bei der der Luftspalt (74) ferner eine Länge aufweist, die gleich einem vorbestimmten minimalen Abstand zwischen dem Auslass (70) und den Fluss-Umlenker (72) ist, wobei der Auslass (70) einen nach unten gerichteten Auslass (80) und einen Durchmesser hat und der Fluss-Umlenker (72) einen oberen Rand hat,
wobei die Länge des Luftspaltes (74) zwischen dem Ausgang (80) des Auslasses (70) und dem oberen Rand des Fluss-Umlenkers (72) gemessen ist, und
wobei die Länge des Lufspaltes (74) größer oder gleich dem Dreifachen des Durchmessers des Auslasses ist.

4. Endoskop-Wiederaufbereitungseinrichtung (30) nach Anspruch 3, die ferner ein Zuführrohr (84) umfasst, welches Stromaufwärts von dem Auslass und mit diesem in Fluidkommunikation verbunden ist, und wobei das Zuführrohr einen Durchmesser aufweist und die Länge des Luftspalts größer oder gleich dem Dreifachen des Durchmessers des Zuführrohres ist.

5. Endoskop-Wiederaufbereitungseinrichtung (30) nach Anspruch 1, bei der die von dem Auslass ausgelassene Flüssigkeit eine erste Flüssigkeit umfasst und die Vorrichtung ferner folgendes umfasst:
c. Mittel (88, 96, 124) zum Auslassen einer zweiten Flüssigkeit in die erste Flüssigkeit in dem Bereich des Fluss-Umlenkers (72), sodass die erste und die zweite Flüssigkeit gemischt werden.

6. Endoskop-Wiederaufbereitungseinrichtung (30) nach Anspruch 5, die ferner folgendes umfasst:
d. Mittel (88, 98, 126) zum Auslassen einer dritten Flüssigkeit in die erste Flüssigkeit in dem Bereich des Fluss-Umlenkers (72), sodass die erste, die zweite und die dritte Flüssigkeit gemischt werden.

7. Endoskop-Wiederaufbereitungseinrichtung (30) nach Anspruch 1, bei der die Auslassrichtung des Auslasses (70) um nicht mehr als 15° von der Vertikalen abweicht.

8. Endoskop-Wiederaufbereitungseinrichtung (30) nach Anspruch 1, bei der der Fluss-Umlenker (72) eine konkave Oberfläche (78) aufweist, die den Strom umlenkt.

9. Endoskop-Wiederaufbereitungseinrichtung (30) nach Anspruch 8, die ferner mindestens eine Leitung (88, 124, 126) zum Einlassen einer flüssigen Chemikalie in den Fluss-Umlenker (72) umfasst, um die mindestens eine flüssige Chemikalie mit dem Wasserstrom zu mischen.

10. Endoskop-Wiederaufbereitungseinrichtung (30) nach Anspruch 9, bei der die mindestens eine Leitung (88) aus einer Oberfläche (78) des Fluss-Umlenkers (72) austritt.

11. Endoskop-Wiederaufbereitungseinrichtung (30) nach Anspruch 9, bei der die mindestens eine Leitung (88) in mindestens einem Auslass zur Zufuhr von (124) flüssigen Chemikalien ausgebildet ist, der oberhalb des Fluss-Umlenkers (72) angeordnet ist.

12. Endoskop-Wiederaufbereitungseinrichtung (30) nach Anspruch 11, bei der der mindestens eine Auslass (124, 126) für die Zufuhr von Chemikalien einen Fluss unter einem Winkel zu dem Wasserfluss ausgibt.

13. Endoskop-Wiederaufbereitungseinrichtung (30) nach Anspruch 11, bei der der mindestens eine Auslass (124, 126) zur Ausgabe von Chemikalien in einem Abstand von dem Wasser-Auslass (70) angeordnet ist, der größer oder gleich dem Dreifachen des Durchmessers des Wasserauslasses ist.

14. Endoskop-Wiederaufbereitungseinrichtung (30) nach Anspruch 3 oder 13, bei der der Durchmesser ein Innendurchmesser oder eine Außendurchmesser ist.

15. Verfahren zum Verhindern eines Rückflusses und zum Richten einer Flüssigkeit in ein Endoskop-Wiederaufbereitungseinrichtungs-Becken (31, 32), das die folgenden Schritte umfasst:
a. Auslassen einer Flüssigkeit aus einem Auslass (70) in eine Abwärtsrichtung;
b. Empfangen der von dem Auslass (70) ausgelassenen Flüssigkeit in einem Fluss-Umlenker (72), der von dem Auslass (70) beabstandet ist,
c. Umlenken der Flüssigkeit in eine vorbestimmte Richtung, die eine wesentliche horizontale Komponente aufweist, in das Endoskop-Wiederaufbereitungseinrichtungs-Becken (31, 32).

## Revendications

1. Dispositif de retraitement d'endoscope (30) comprenant un bassin de retraitement d'endoscope (31, 32) et un appareil (60, 60') destiné à fournir de l'eau au bassin de retraitement d'endoscope (31, 32) tout en évitant des interférences avec une zone de travail du bassin (31, 32), l'appareil étant **caractérisé par** :
a. une goulotte (70) évacuant sélectivement un liquide dans une direction verticale vers le bas ; et
b. un déflecteur (72) séparé de la goulotte (70) par un intervalle d'air (74) et positionné sous la goulotte (70) et recevant le liquide évacué par la goulotte (70) et redirigeant le liquide évacué dans une direction qui n'est pas verticale dans le bassin d'endoscope (31, 32).

2. Dispositif de retraitement d'endoscope (30) selon la revendication 1, dans lequel le déflecteur comprend une surface (78) redirigeant le liquide évacué dans une direction ayant une composante horizontale et une composante verticale.

3. Dispositif de retraitement d'endoscope (30) selon la revendication 1, dans lequel l'intervalle d'air (74) a en outre une longueur égale à une distance minimum prédéterminée entre la goulotte (70) et le déflecteur (72), dans lequel la goulotte (70) a un accès de sortie dirigé vers le bas (80) et un certain diamètre et le déflecteur (72) a un bord supérieur,
dans lequel la longueur de l'intervalle d'air (74) est mesurée entre l'accès de sortie (80) de la goulotte (70) et le bord supérieur du déflecteur (72),
et dans lequel la longueur de l'intervalle d'air est égale ou supérieure à trois fois le diamètre de la goulotte.

4. Dispositif de retraitement d'endoscope (30) selon la revendication 3, comprenant en outre un tuyau d'alimentation (84) en amont de la goulotte et en communication de fluide avec celle-ci, et dans lequel le tuyau d'alimentation a un certain diamètre et la longueur de l'intervalle d'air est égale ou supérieure à trois fois le diamètre du tuyau d'alimentation.

5. Dispositif de retraitement d'endoscope (30) selon la revendication 1, dans lequel le liquide évacué par la goulotte comprend un premier liquide et l'appareil comprend en outre :
c. des moyens (88, 96, 124) d'évacuation d'un deuxième liquide dans le premier liquide dans la région du déflecteur (72) de telle sorte que les premier et deuxième liquides sont mélangés.

6. Dispositif de retraitement d'endoscope (30) selon la revendication 5, comprenant en outre :
d. des moyens (88, 98, 126) d'évacuation d'un troisième liquide dans le premier liquide dans la région du déflecteur (72) de telle sorte que les premier, deuxième et troisième liquides sont mélangés.

7. Dispositif de retraitement d'endoscope (30) selon la revendication 1, dans lequel la direction d'évacuation de la goulotte (70) ne fait pas un angle supérieur à quinze degrés par rapport à la verticale.

8. Dispositif de retraitement d'endoscope (30) selon la revendication 1,
dans lequel le déflecteur (72) a une surface concave (78) redirigeant le flux.

9. Dispositif de retraitement d'endoscope (30) selon la revendication 8, comprenant en outre au moins un passage (88, 124, 126) pour fournir un produit chimique liquide au déflecteur (72) pour mélanger ledit au moins un produit chimique liquide au flux d'eau.

10. Dispositif de retraitement d'endoscope (30) selon la revendication 9, dans lequel ledit au moins un passage (88) débouche dans une surface (78) du déflecteur (72).

11. Dispositif de retraitement d'endoscope (30) selon la revendication 9, dans lequel ledit au moins un passage est dans au moins une goulotte de fourniture de produit chimique liquide (124) au-dessus du déflecteur (72).

12. Dispositif de retraitement d'endoscope (30) selon la revendication 11, dans lequel ladite au moins une goulotte de fourniture de produit chimique (124, 126) émet un flux avec un angle vers le flux d'eau.

13. Dispositif de retraitement d'endoscope (30) selon la revendication 11, dans lequel ladite au moins une goulotte de fourniture de produit chimique (124, 126) est séparée d'une distance par rapport à la goulotte d'eau (70) égale ou supérieure à trois fois le diamètre de la goulotte d'eau.

14. Dispositif de retraitement d'endoscope (30) selon la revendication 3 ou 13, dans lequel le diamètre est un diamètre intérieur ou un diamètre extérieur.

15. Procédé pour empêcher un retour de flux et pour diriger un liquide dans un bassin de retraitement d'endoscope (31, 32) comprenant les étapes suivantes :
a. évacuer un liquide à partir d'une goulotte (70) dans une direction vers le bas ;
b. recevoir le liquide évacué par la goulotte (70) dans un déflecteur (72) distant de la goulotte (70) ; et
c. rediriger le liquide dans une direction prédéterminée ayant une composante sensiblement horizontale dans le bassin de retraitement d'endoscope (31, 32).
